# EUROPEAN PATENT APPLICATION

(11) **EP 4 652 945 A1**
(43) Date of publication of application: **26.11.2025**
(21) Application number: 24401017.9
(22) Date of filing: 23.05.2024
(51) Int. Cl.: A61B 17/72

(54) **BONE TRANSPORT SYSTEM**

(71) Applicant: Vogt, Dennis, 10249 Berlin (DE); Unamuno Eguren, Anartz, 01109 Dresden (DE)
(72) Inventor: VOGT, Dennis, 10249 Berlin (DE)
(74) Representative: Galbaian S.Coop.

(57) **Abstract**

The invention relates to a bone transport system for a bone (4) comprising a first bone portion (4.4), a second bone portion (4.5) and at least a first bone segment (4.1) arranged between both bone portions (4.4, 4.5). The system (100) comprises a hollow carrier (3) configured for being secured to at least one of the bone portions (4.4, 4.5); at least one displaceable deflection element (6) to be arranged inside the carrier (3); a first tension line (5.1) associated with the displaceable deflection element (6) and secured to the first bone segment (4.1); and a driving line (7) associated with the displaceable deflection element (6) such that it causes the displacement of said displaceable deflection element (6) when pulled or pushed thanks to its association with the first tension line (5.1).

## Description

### TECHNICAL FIELD

The present invention relates to bone transport systems.

### PRIOR ART

There are injuries or diseases that result in a bone defect, i.e. a piece or segment of bone is lost, leaving a bone gap or a bone defect. This can happen, for example, as a result of road traffic accidents or war injuries.

In the context of stabilisation/trauma surgery, bone healing may be impaired, or inflammation may spread to the bone, necessitating bone resection. Malignant bone neoplasms, i.e. tumours, are also associated with extended bone segment resections.

These gaps or defects lead to an unstable limb. The bone cannot bear weight. Patients are often confined to walking aids or a wheelchair and are unable to work. Complex procedures are carried out in an attempt to close these gaps and restore a fully load-bearing bone without producing more loss of length in this bone or the limb in question.

Several procedures have been established to close these gaps. The most important one includes direct filling of short defects with the patient's own bone, which is harvested from another site. Then there is a two-stage procedure in which a spacer is inserted before the filling, which then leads to the formation of a membrane as part of a foreign body reaction, which can then be filled with the patient's own bone once the spacer has been removed. This technique allows longer defect distances to be overcome.

An old procedure is known as segment transport. In this procedure, the same bone is cut at a different location in order to transport the resulting free segment over the defect area. Under suitable transport conditions, new bone forms in the gap that opens up at the cut/osteotomy site. This allows considerable defect distances to be overcome.

The original segment transport procedure was linked to an external transport frame, an external fixator, in particular a ring fixator. There are now systems that enable internal transport without an external scaffold, by means of a magnet or induction (electric) for example. This has considerably simplified treatment for patients. The transport speed at an osteotomy site is independent of whether an external or internal method is selected for transport.

Most common internal segment transports are primarily configured for, and limited to, transporting one segment, as the one described in US RE49,061 E. The solution described in said document includes a complete kit to be housed in the intramedullary housing of the bone and attached to the bone, and said kit comprises a plurality of elements including a magnetic assembly and a telescopic nail, that makes possible the transport of one bone segment a certain length.

### DISCLOSURE OF THE INVENTION

The object of the invention is to provide a bone transport system, as defined in the claims.

The system is adapted for a bone of a patient comprising a first bone portion, a second bone portion, and a first bone segment arranged between both bone portions and physically separated from said bone portions, and for filling with new bone a defect of a certain length upon the transport of said first bone segment between both bone portion.

The bone transport system comprises an intramedullary hollow carrier opened at both ends and comprising a longitudinal axis, configured for being secured to at least one of the first bone portion and the second bone portion, preferably to both bone portions, when housed in the bone as an internal skeleton, such that said carrier remains static.

The system further comprises a first displaceable deflection element to be arranged inside the carrier and displaceable along the longitudinal axis of said carrier; a first tension line that in use is associated with the first displaceable deflection element and secured to the first bone segment, such that said bone segment is transported when the first displaceable deflection element is displaced; and a driving line which in use is associated with the first displaceable deflection element and configured to cause the displacement of the first displaceable deflection element along the longitudinal axis of the carrier when actuated. The bone segment is then transported as a result of the displacement of the displaceable deflection element.

The proposed system acts then a pulley-cable (line) based system, based at least on the principles of a loose pulley system, resulting in a simple and modular transport system. This makes it easy for being integrated into a bone and easy to manipulate and also provides a flexibility related to the length to be transported by the bone segment as a result of an actuation on the driving line, at a low financial cost compared with the solutions of the prior art. Known devices facing the callus distraction are limited in transport range, speed of reconstruction and quantity of transport segments, and the proposed solution solves these limitations thanks to their simple and flexible configuration.

These and other advantages and features of the invention will become apparent in view of the figures and the detailed description of the invention.

### DESCRIPTION OF THE DRAWINGS

Figure 1 shows an embodiment of the bone transport system according to the invention, configured for transporting a single bone segment.
Figure 2 shows the bone transport system of Figure 1, integrated into a bone.
Figure 3a shows another embodiment of the bone transport system according to the invention, configured for transporting a single bone segment, and integrated into a bone.
Figure 3b shows another embodiment of the bone transport system according to the invention, configured for transporting a single bone segment, and integrated into a bone.
Figure 4 shows another embodiment of the bone transport system according to the invention, configured for transporting a single bone segment, and integrated into a bone.
Figure 5 shows another embodiment of the bone transport system according to the invention, configured for transporting a single bone segment, and integrated into a bone.
Figure 6 shows an embodiment of the bone transport system according to the invention, configured for transporting a plurality of bone segments in a same direction, and integrated into a bone.
Figure 7 shows another embodiment of the bone transport system according to the invention, configured for transporting a plurality of bone segments in a same direction, and integrated into a bone.
Figure 8 shows another embodiment of the bone transport system according to the invention, configured for transporting a plurality of bone segments in opposite directions, and integrated into a bone.

### DETAILED DISCLOSURE OF THE INVENTION

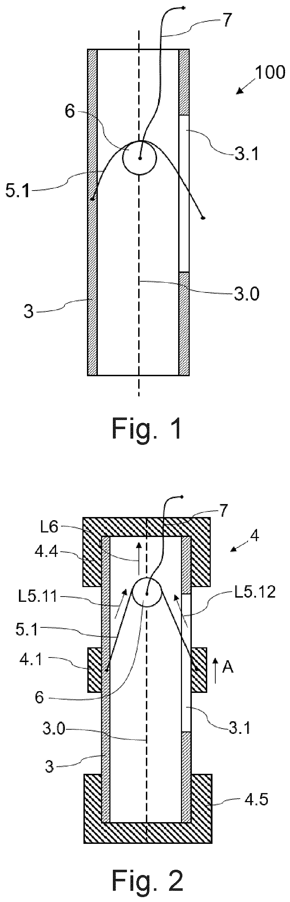

The bone transport system 100, as the embodiment shown in Figure 1, is adapted for transporting at least one bone segment 4.1 of a bone 4 comprising a first bone portion 4.4 (proximal portion) and a second bone portion 4.5 (distal portion). Said bone segment 4.1 is arranged between said two bone portions 4.4 and 4.5 and physically separated from said two bone portions 4.4 and 4.5, and the system 100 causes the controlled transport of the bone segment 4.1 between the bone portions 4.4 and 4.5, a defect of a certain length present between said bone portions 4.4 and 4.5 being filled with new bone as a result.

The system 100 comprises an intramedullary hollow carrier 3 configured for being secured to at least one of the bone portions 4.4 and 4.5, preferably to both bone portions 4.4 and 4.5, and preferably forming a single body, said carrier 3 remaining static during the use of the system 100 for filling with new bone the defect present in the bone 4.

The carrier 3 is housed in the bone 4, preferably traversing said portions 4.4 and 4.5 as shown in Figure 2 (and also the bone segment 4.1), comprises a main longitudinal axis 3.0 and is open at its both ends to facilitate any element to be introduced in, or evacuated from, the carrier 3. When transported, the bone segment 4.1 is displaceable along said longitudinal axis 3.0, towards the first bone portion 4.4 or towards the second bone portion 4.5 depending on the configuration of the system 100.

The system 100 also comprises at least a first displaceable deflection element 6 arranged, in use, inside the hollow carrier 3, and displaceable along the longitudinal axis 3.0 of said carrier 3, the bone segment 4.1 being transported as a result of the displacement of said displaceable deflection element 6 as it is detailed hereinafter. Preferably the displaceable deflection element 6 is a pulley.

The system 100 further comprises a first tension line 5.1 that, in use, is secured to the bone segment 4.1 and is associated with the first displaceable deflection element 6, and a driving line 7 associated with the first displaceable deflection element 6 and configured to cause the displacement of the first displaceable deflection element 6 along the longitudinal axis 3.0 of the carrier 3 when actuated. Then, when the driving line 7 is actuated the first displaceable deflection element 6 is displaced along said longitudinal axis 3 a required length, and due to the association of said first displaceable deflection element 6 with the first tension line 5.1, said displacement also causes the transport or displacement of the bone segment 4.1.

In some embodiments of the invention, the first tension line 5.1 is also fixed to a static part of the system 100 (to the carrier 3 for example, as shown in the embodiment of Figure 2) or to a static part of the bone 4 (to a bone portion 4.4 or 4.5), for example. In said embodiments the first tension line 5.1 is partially wrapped around the first displaceable deflection element 6, the displacement of said first displaceable deflection element 6 pushing said first tension line 5.1 and then the transport or displacement of the bone segment 4.1 being caused.

In the embodiment shown in Figure 2, as the first tension line 5.1 is wrapped around the displaceable deflection element 6, the length transported by the bone segment 4.1 is increased compared to a system 100 where a tension line 5.1 does not wrap any displaceable deflection element 6, for the same action upon the driving line 7. When the driving line 7 is pulled a specific length, the displaceable deflection element 6 travels said length (referenced with arrow L6 in Figure 2) and this displacement causes the first tension line 5.1 to also travel said length on each side of the wrapped displaceable deflection element 6 (referenced in said Figure 2 with arrows L5.11 and L5.12), said first tension line 5.1 traveling said length twice. Said double travel is transferred to the bone segment 4.1, the length transported by said bone segment 4.1 being then twice the length travelled by the driving line 7. In Figure 2 the direction of the displacement of the displaceable deflection element 6 and the bone segment 4.1 is referenced with an arrow A.

Upon an action of the driving line 7, for a given time, if the length transported by a bone segment 4.1 is increased, this means that the transport speed of said bone segment 4.1 has also been increased. Therefore, any reference to the length transported by a bone segment 4.1 made along the description is also valid for the speed of said bone segment 4.1 and, likewise, any reference to the speed of a bone segment 4.1 made along the description is also valid for the length transported by said bone segment 4.1.

In the context of the invention the expression "wrapped around" or "wrapping" must be understood as being partially wrapped around, as the case of the tension line 5 in respect of the displaceable deflection element 6 of the embodiment shown in Figure 2 (in said Figure 2 the tension line 5.1 wraps over the displaceable deflection element 6, but of course in other embodiments or configurations it could wrapping under said displaceable deflection element 6).

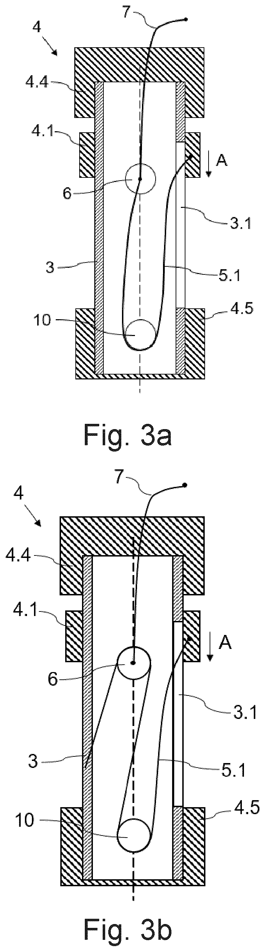

In some embodiments the system 100 comprises at least one fixed deflection element 10, preferably a pulley, to be associated to the carrier 3, to one of the bone portions 4.4 and 4.5 or to any other static part, in a non-displaceable manner. In these embodiments, the first tension line 5.1 is fixed to the bone segment 4.1, and also to a static point of the system 100 (as in the embodiment of Figure 3b, where it is fixed to the carrier 3 of the system), to a static part of the bone 4 or to a displaceable point of the system 100 (the first displaceable deflection element 6 in the embodiment of Figure 3a).

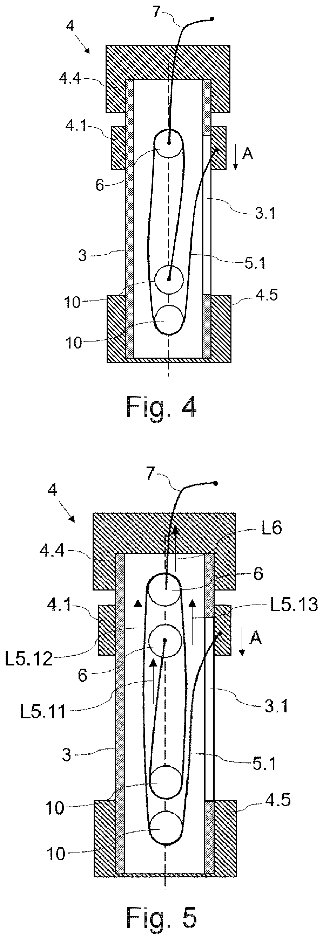

A fixed deflection element 10 can be used as a fixed point for a tension line 5.1, to guide the first tension line 5.1 to the required place (to the bone segment 4.1 for example) and/or to increase the length transported by the corresponding bone segment 4.1 as it will be explained in more detail later, so the system 100 allows to increase the length to be transported by the bone segment 4.1 (speed), in each actuation upon it, in an easy way if so required (as it will be described in respect of the embodiment of Figure 5 for example).

According to the embodiment shown in Figure 3a, the fixed deflection element 10 guides the tension line 5.1 towards the bone segment 4.1 and does not cause any increase of the length transported by the bone segment 4.1. The first tension line 5.1 does not wrap the displaceable deflection element 6, therefore, said first tension line 5.1 only travels the same length as the displaceable deflection element 6, and in said configuration the length transported by the bone segment 4.1 is not increased (in contrast to the configuration of Figure 2).

In the embodiment shown in Figure 3b, the first tension line 5.1 is fixed to the bone segment 4.1 and also to a static part of the system 100 (the carrier 3 in this case). Said tension line 5.1 also wraps the displaceable deflection element 6, therefore, the displacement of said displaceable deflection element 6 causes the same effect over the first tension line 5.1 as for the embodiment of Figure 2, the length transported by a bone segment 4.1 with the embodiment of Figure 3b being twice the length transported by a bone segment 4.1 with the embodiment of Figure 3a.

In some embodiments of the system 100 with one displaceable deflection element 6, as the one shown in Figure 4, the system 100 comprises a plurality of fixed deflection elements 10 arranged in series along the longitudinal axis 3.0 of the carrier 3 (two in said embodiment, an uppermost fixed deflection element 10 and a lowermost fixed deflection element 10). One end of the first tension line 5.1 is fixed to the uppermost fixed deflection element 10, wraps the displaceable deflection element 6 and the lowermost deflection element 10, and it is guided towards the first bone segment 4.1 by said lowermost deflection element 10. as the tension line 5.1 wraps the first displaceable deflection element 6, the displacement of said displaceable deflection element 6 causes the same effect over the first tension line 5.1 as for the embodiments of Figure 2 and 3b, the length transported by a bone segment 4.1 with the embodiment of Figure 4 being the same as for said embodiments of Figure 2 and 3b. The difference between the embodiments of Figure 3b and Figure 4 is then that, although in both cases the first tension line 5.1 is fixed to a static part of the system 100, said first tension line 5.1 is fixed to the carrier 3 in the embodiment of Figure 3b and to a fixed deflection element 10 in the embodiment of Figure 4.

In order to facilitate the adding of more deflection elements 10 linked to the tension line 5.1 in said manner, or if so required, more displaceable deflection elements 6 can also be included in series arrangement along the longitudinal axis 3.0 as shown for example in the embodiment of Figure 5.

When the system 100 comprises a plurality of displaceable deflection elements 6, said displaceable deflection elements 6 are preferably associated to each other such that an actuation on the driving line 7 causes all the displaceable deflection elements 6 to displace together.

In the embodiment shown in Figure 5, the system 100 comprises two displaceable deflection elements 6 arranged in series along the longitudinal axis 3.0 of the carrier 3 and two fixed deflection elements 10 also arranged in series along said longitudinal axis 3.0. One end of the first tension line 5.1 is fixed to the lowermost displaceable deflection element 6 and the other end is fixed to the bone segment 4.1, the lowermost fixed deflection element 10 guiding said tension line 5.1 towards the bone segment 4.1.

The first tenson line 5.1 is extended from the lowermost displaceable deflection element 6 towards the uppermost fixed deflection element 10, wraps said uppermost fixed deflection element 10 from where it is guided towards the uppermost displaceable deflection element 6, wraps said uppermost displaceable deflection element 6 from where it is guided towards the lowermost fixed deflection element 10, wraps said lowermost fixed deflection element 10 and is guided towards the bone segment 4.1. When the displaceable deflection elements 6 are displaced a corresponding length in the direction L6, the first tension line 5.1 travels also said length three times as denoted with arrows L5.11, L5.12 and L5.13. Said three travels are reflected in the transport o the bone segment 4.1, the length transported by said bone segment 4.1 being three times the length displaced by the displaceable deflection elements 6 (by the driving line 7).

Therefore, as already stated, increasing the length transported by a bone segment 4.1 by a same actuation on the driving line 7 is very easy with the proposed system 100, just adding as many displaceable deflection elements 6 and/or deflection elements 10 as required, and linked to the tension line 5.1 fixed to said bone segment 4.1 as required, and lengthening the path of the first tension line 5.1 in order to cause said first tension line 5.1 to cooperate with the required deflection elements 6 and 10 of the corresponding embodiment of the system 100. Summing up, the system 100 takes advantage of the block and tackle principle to increase the length transported by the bone segment 4.1 as required, said system 100 being very flexible and easy to implement.

The system 100 can also be adapted easily for transporting a plurality of bone segments 4.1 and 4.2 of the bone 4 simultaneously (as many bone segments 4.1 and 4.2 as required), said bone segments 4.1 and 4.2 being arranged in series between the bone portions 4.4 and 4.5. In said embodiments the system 100 comprises a respective tension line 5.1 and 5.2 for each bone segment 4.1 and 4.2 to be transported, each tension line 5.1 and 5.2 being fixed to a respective bone segment 4.1 and 4.2.

All the tension lines 5.1 and 5.2 are associated to at least one displaceable deflection element 6 such than the displacement of said displaceable deflection element 6 affects the tension line 5.1 and 5.2 causing the transport of the bone segments 4.1 and 4.2. The system 100 allows to associate each tension line 5.1 and 5.2 to the required displaceable deflection elements 6, it being also possible for any displaceable deflection element 6 of the system 100 to be associated to only one tension line 5.1 or 5.2 of said system 100, to a plurality of tension lines 5.1 and 5.2 or to all the tension lines 5.1 and 5.2. Where there is more than one displaceable deflection element 6, said displaceable deflection elements 6 are arranged in series along the longitudinal axis 3.0 of the carrier 3, inside said carrier 3, and preferably linked to each other such that they are displaced together as mentioned previously.

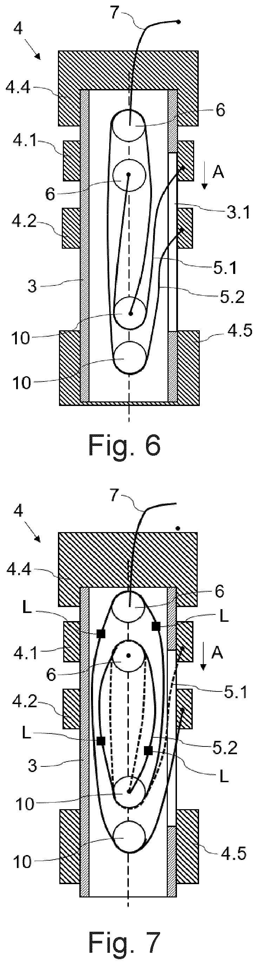

In the embodiments of the system 100 adapted for transporting a plurality of bone segments 4.1 and 4.2 in the same direction, the system 100 is preferably configured to transport the bone segments 4.1 and 4.2 at different speeds, the speed of a bone segment 4.2 being higher than the speed of the preceding bone segment 4.1. With the bone segments 4.1 and 4.2 being transported at different speeds, the system 100 comprise, at least, as many deflection elements 10 as different speeds are required, and each tension line 5.1 and 5.2 linked to a specific speed is wrapped around a specific fixed deflection element 10 as shown for example in Figures 6 and 7. Again, as described for the embodiments where only one bone segment 4.1 is transported, the speed of a bone segment 4.1 or 4.2 can be increased using more deflection elements 6 and 10 together with a specific link between the corresponding tension line 5.1 and 5.2 and said deflection elements 6 and 10, and lengthening the path of the corresponding tension line 5.1 and 5.2.

The first tension line 5.1 of the embodiment shown in Figure 6 is fixed to the lowermost displaceable deflection element 6 and wraps the uppermost fixed deflection element 10 to be guided to the first bone segment 4.1 to which it is also attached. As said tension line 5.1 does not wrap any displaceable deflection element 6, the same effect on the first bone segment 4.1 is achieved as for the bone segment 4.1 of the embodiment of Figure 3a.

The second tension line 5.2 of the embodiment shown in Figure 6 is fixed to the uppermost fixed deflection element 10, wraps the uppermost displaceable deflection element 6 and also wraps the lowermost fixed deflection element 10 to be guided to the second bone segment 4.2 to which it is also attached. As said second tension line 5.2 wraps the uppermost displaceable deflection element 6, the same effect on the second segment 4.2 is achieved as for the bone segment 4.1 of the embodiment of Figure 4.

The first tension line 5.1 of the embodiment shown in Figure 7 is fixed to the uppermost fixed deflection element 10, wraps the lowermost displaceable deflection element 6 and also wraps the uppermost fixed deflection element 10 to be guided to the first bone segment 4.1 to which it is also attached. As said first tension line 5.1 wraps the lowermost displaceable deflection element 6, the same effect on the first bone segment 4.1 is achieved as for the second bone segment 4.1 of the embodiment of Figure 6.

The second tension line 5.2 of the embodiment shown in Figure 7 is fixed to the uppermost fixed deflection element 10, wraps the lowermost displaceable deflection element 6, wraps the uppermost fixed deflection element 10, wraps the uppermost displaceable deflection element 6 and also wraps the lowermost fixed deflection element 10 to be guided to the second bone segment 4.2 to which it is also attached. Said second tension line 5.2 wraps both displaceable deflection elements 6, therefore, four sections of said second tension line 5.2 travels the same length as said displaceable deflection elements 6 are displaced and the length transported by the second bone segment 4.2 is equal to four times the length displaced by said displaceable deflection elements 6. Said sections of the second tension lines 5.2 are pointed out by a respective black square L in Figure 7.

Hence, in the embodiment shown in Figure 7 the first bone segment 4.1 is transported at a first speed (equal to the second speed of the second bone segment 4.2 of the embodiment of Figure 6) and the second bone segment 4.2 is transported at a second speed (the second speed doubling the first speed).

In some of the embodiments of the system 100 adapted for transporting a plurality of bone segments 4.1 and 4.2, as the ones shown in Figure 6 and 7, the system 100 is adapted for transporting all the bone segments 4.1 and 4.2 in the same direction A.

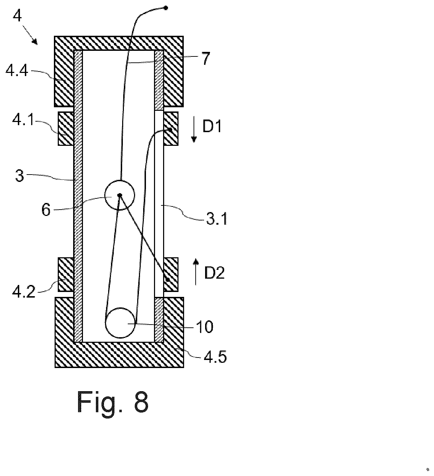

In other embodiments adapted for transporting a plurality of bone segments 4.1 and 4.2, as the one shown in Figure 8, the system 100 is adapted for transporting at least one bone segment 4.1 in a first direction D1 and for transporting at least another bone segment 4.2 in a second direction D2 opposite the first direction D1. Both bone segments 4.1 and 4.2 can be transported at a same speed or even at different speeds. If more than one bone segment is transported in the same direction, the previously given explanations are also valid for said bone segments.

In some embodiments not shown in figures, the system 100 comprises a driving unit (a magnetic motor for example) and said driving unit can be inside the hollow carrier 3 or outside the hollow carrier 3.

In other embodiments, the system 100 does not comprise any driving unit and the driving line comprises an end that is arranged outside the carrier 3 in order to be accessible from the exterior of the bone 4, once the system 100 is integrated in the corresponding patient, such that said end can be easily connected to a driving unit disposed outside the bone (in another bone 4 or outside the patient), or, even, it can be easily actuated manually if so required when accessible from the exterior of the patent.

In some embodiments the system 100 further comprises a driving deflection element not shown in figures, preferably a pulley, arranged inside the carrier 3 and associated to said carrier 3 or to one of the bone portions 4.4 and 4.5 in a displaceable fixed manner in use. The driving line 7 wraps said driving deflection element, and the carrier 3 comprises preferably an opening for being traversed by said driving line 7 and facilitating said driving line 7 to exit the carrier 3. Thanks to the driving deflection element the driving line 7 can be guided towards the required place easily.

The carrier 3 can comprise at least one opening 3.1 being traversed by at least the tension lines 5.1 and 5.2 comprised by the system 100 in any of its embodiments, in order to facilitate the connection of said tension lines 5.1 and 5.2 to the corresponding bone segment 4.1 and 4.2. The carrier 3 could also comprise at least one hole or slot 3.1 to be traversed by the driving line 7, when there is no actuator inside the carrier 3. Preferably, the carrier 3 comprises a single opening for all the required lines, but it could also comprise a respective opening 3.1 for each of said lines or as many openings 3.1 as required.

When the carrier 3 comprises a single opening 3.1, the length of said opening 3.1 is equal or greater than the length of the defect to be filled with new bone, in order to allow the lines to exit the carrier easily 3 along all the range of the transport length. When the carrier 3 comprises a plurality of openings 3.1 they could be arranged in different axial positions in respect of the longitudinal axis 3.0 of the carrier 3, and the length of each opening 3.1 should assure the transport length of the associated bone segment 4.1 and 4.3 or the displaceable length of the associated displaceable deflection element 6.

## Claims

1. Bone transport system for a bone (4) of a patient comprising a first bone portion (4.4) a second bone portion (4.5) and a first bone segment (4.1) arranged between both bone portions (4.4, 4.5) and physically separated from said bone portions (4.4, 4.5), and for filling with new bone a defect of a certain length upon the transport of said first bone segment (4.1), **characterized in that** the bone transport system (100) comprises an intramedullary hollow carrier (3) opened at both ends and comprising a main longitudinal axis (3.0), said carrier (3) being secured to at least one of the first bone portion (4.4) and the second bone portion (4.5), preferably to both bone portions (4.4, 4.5), in use; a first displaceable deflection element (6), preferably a pulley, which, in use, is arranged inside the carrier (3) and is displaceable along the longitudinal axis (3.0) of said carrier (3); a first tension line (5.1) which, in use, is secured to the first bone segment (4.1) and associated with the first displaceable deflection element (6) such that said bone segment (4.1) is transported when the first displaceable deflection element (6) is displaced; and a driving line (7) which, in use, is associated with the first displaceable deflection element (6) and configured to cause the displacement of the first displaceable deflection element (6) along the longitudinal axis (3.0) of the carrier (3) when actuated.

2. Bone transport system according to claim 1, comprising at least one fixed deflection element (10), preferably a pulley, which, in use, is arranged inside the carrier (3), associated to said carrier (3) or to one of the bone portions (4.4, 4.5) in a non-displaceable manner, and configured to deflect the path of said first tension line (5.1).

3. Bone transport system according to claim 2, comprising at least one additional fixed deflection element (10), preferably a pulley, which, in use, is arranged inside the carrier (3), associated to said carrier (3) or to one of the bone portions (4.4, 4.5) in a non-displaceable manner, arranged in series with the other fixed deflection element (10) along the longitudinal axis (3.0) of said carrier (3), and configured deflect the path of the first tension line (5.1).

4. Bone transport system according to any of claims 1 to 3, wherein in use the first tension line (5.1) is also secured to the first displaceable deflection element (6).

5. Bone transport system according to any of claims 1 to 4, comprising at least one additional displaceable deflection element (6) to be arranged inside the carrier (3) in series with the first displaceable deflection element (6) along the longitudinal axis (3.0) of said carrier (3), in use, the additional displaceable deflection element (6) being configured to be displaceable along said longitudinal axis (3.0) by the actuation of the driving line (7) together with the first displaceable deflection element (6), and the first tension line (5.1) wrapping the additional displaceable deflection element (6).

6. Bone transport system according to any of claims 1 to 4, wherein the system (100) is adapted for a bone (4) comprising the first bone segment (4.1) and at least one additional bone segment (4.2) arranged in series between both bone portions (4.4, 4.5), and for filling with new bone a defect of a certain length upon the transport of said bone segments (4.1, 4.2), the system (100) comprising an additional tension line (5.2) for each additional bone segment (4.2), each additional tension line (5.2) being, in use, secured to the corresponding additional bone segment (4.2) and associated to at least one displaceable deflection element (6) such that said bone segment (4.1) is transported when said displaceable deflection element (6) is displaced.

7. Bone transport system according to claim 6, comprising at least one fixed deflection element (10), preferably a pulley, for each of the tension lines (5.1, 5.2), each tension line (5.1, 5.2) wrapping the corresponding fixed deflection element (10) in use.

8. Bone transport system according to claim 7, wherein in use each additional tension line (5.2) is secured to a displaceable deflection element (6), to a fixed deflection element (10), to the carrier (3) or to a bone portion (4.4, 4.5).

9. Bone transport system according to any of claims 6 to 8, wherein the system (100) is adapted for transporting all the bone segments (4.1, 4.2) in the same direction (A).

10. Bone transport system according to any of claims 6 to 8, wherein the system (100) is adapted for transporting at least one bone segment (4.1) in a first direction (D1) and for transporting at least another bone segment (4.2) in a second direction (D2) opposite the first direction (D1).

11. Bone transport system according to any of claims 1 to 10, comprising a driving unit for being secured to the driving line (7) and for causing the displacement of the displaceable deflection elements (6) when actuated, said driving unit preferably comprising a motor.

12. Bone transport system according to claim 10 or 11, wherein the driving line (7) is associated with one displaceable deflection element (6) and is also connected to the driving unit in use, the driving line (7) being then configured to be accessible from outside the bone (4) in order to be actuated from the exterior of said bone (4), the driving unit being configured to be arranged outside the carrier (3).

13. Bone transport system according to claim 12, comprising a driving deflection element, preferably a pulley, arranged inside the carrier (3) and associated to said carrier (3) or to one of the bone portions (4.4, 4.5) in a displaceable fixed manner in use, the driving line (7) wrapping said driving deflection element, the carrier (3) comprising preferably an opening for being traversed by said driving line (7).

14. Bone transport system according to any of claims 1 to 13, wherein the carrier (3) comprises at least one opening (3.1) for being traversed at least by the tension lines (5.1, 5.2) of the system (100), or a given opening for each of said tension lines (5.1, 5.2).

15. Bone transport system according to claim 14, wherein the carrier (3) comprises at least one opening (3.1) for all the tension lines (5.1, 5.2) of the system (100), the length of the one opening (3.1) along the longitudinal axis (3.0) of said carrier (3) being at least equal to the certain length of the defect to be filled with new bone.
